(19) Europäisches Patentamt
European Patent Office
Office européen des brevets

(11) Numéro de publication : 0 426 562 B1

# (12) FASCICULE DE BREVET EUROPEEN

(45) Date de publication du fascicule du brevet : **16.03.94 Bulletin 94/11**

(51) Int. Cl.$^5$ : **C07D 211/14,** A61K 31/445

(21) Numéro de dépôt : **90403078.0**

(22) Date de dépôt : **30.10.90**

## (54) Dérivés de 1-(4-aminophényl)-2-pipéridinopropanone, procédé de préparation et utilisation en thérapeutique.

(30) Priorité : **30.10.89 FR 8914217**

(43) Date de publication de la demande : **08.05.91 Bulletin 91/19**

(45) Mention de la délivrance du brevet : **16.03.94 Bulletin 94/11**

(84) Etats contractants désignés : **AT BE CH DE DK ES FR GB GR IT LI LU NL SE**

(56) Documents cités :
**EP-A- 0 138 714**
**EP-A- 0 174 242**

(73) Titulaire : **LABORATOIRE L. LAFON**
**19 Avenue du Professeur Cadiot**
**F-94701 Maisons Alfort (FR)**

(72) Inventeur : **Lafon, Louis**
**5, rue de l'Alboni**
**F-75016 Paris (FR)**

(74) Mandataire : **Le Guen, Gérard et al**
**CABINET LAVOIX 2, place d'Estienne d'Orves**
**F-75441 Paris Cédex 09 (FR)**

EP 0 426 562 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

DOMAINE DE L'INVENTION

La présente invention concerne des dérivés de 1-(4-aminophényl)-2-pipéridinopropanone. Elle concerne également un procédé de préparation de ces produits et leur utilisation en thérapeutique dans le domaine cardiovasculaire.

ART ANTERIEUR

Dans EP-A-0138714 on a décrit des dérivés de 1-(acétylaminophényl)-2-aminopropanone de formule

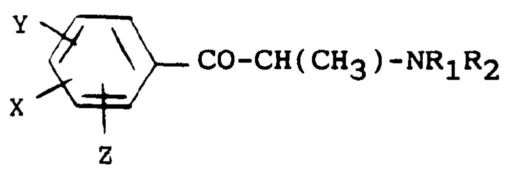 (Io)

dans laquelle notamment
$R_1$ et $R_2$ considérés ensemble peuvent former avec l'atome d'azote auquel ils sont liés un groupe N-hétérocyclique de 5 à 7 sommets susceptible (i) de comporter un second hétéroatome notamment choise parmi N, O et S, et (ii) d'être substitué, ledit groupe hétérocyclique $NR_1R_2$ étant notamment choisi parmi l'ensemble constitué par les groupes pyrrolidino, morpholino, thiomorpholino, pipéridino, hexaméthylèneimino, pipérazino, 4-méthyl-pipérazino, 4(-β-hydroxyéthyl)-pipérazino, 4-phénylpipérazino, 4-(p-chlorophényl)-pipérazino;
et X est $CH_3CONH$ et Y et Z, qui peuvent être identiques ou différents, représentent chacun un atome d'hydrogène ou d'halogène.

Ces composés ont été décrits comme des antidépresseurs du système nerveux central qui peuvent éventuellement présenter des propriétés vasodilatatrices.

Dans EP-A-0 174 242 on a décrit des dérivés de 1-(4-aminophényl)-2-aminopropanone de formule

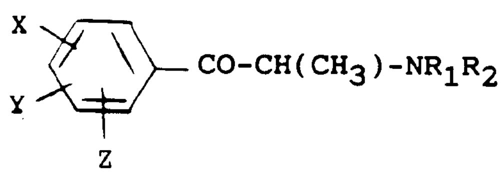

dans laquelle notamment
X est $NH_2$
Y et Z qui peuvent être identiques ou différents représentent un atome d'hydrogène ou d'halogène, et
$R_1$ et $R_2$, considérés ensemble, peuvent former avec l'atome d'azote auquel ils sont liés un groupe N-hétérocyclique de 5 à 7 sommets, pouvant comporter un second hétéroatome choisi parmi N, O et S et pouvant être substitué, ledit groupe hétérocyclique $NR_1R_2$ étant choisi parmi l'ensemble comprenant les groupes pyrrolidino, morpholino, thiomorpholino, pipéridino, hexaméthylèneimino, pipérazino, 4-méthylpipérazino, 4-phénylpipérazino, 4-(2-hydroxyéthyl)-pipérazino, 4-(p-chlorophényl)-pipérazino.

Ces composés ont également été décrits comme des antidépresseurs du système nerveux central qui peuvent éventuellement présenter des propriétés vasodilatatrices.

BUT ET OBJET DE l'INVENTION

Selon l'invention on propose de nouveaux composés appartenant à la famille des dérivés de 1-(aminophényl)-2-aminopropanone et fournit leur procédé de préparation, ces nouveau produits étant particulièrement utiles en thérapeutique.

Ces nouveaux composés se distinguent par des effets vasodilatateurs bénéfiques dans les maladies du système cardiovasculaire, effets dont sont dépourvus les composés les plus voisins décrits dans EP-A-0 174 242 et EP-A-0 138 714.

Les composés selon l'invention sont caractérisés en ce qu'ils sont choisis parmi

(a) les composés répondant à la formule générale

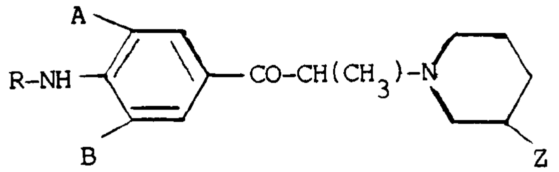

(I)

où R est H ou $CH_3CO$, A est H ou Cl, B indépendamment de A est H ou Cl, et Z est un groupe alkyle en $C_1$-$C_4$; et,

(b) leurs sels d'addition.

DESCRIPTION DETAILLEE DE L'INVENTION

Parmi les groupes alkyle en $C_1$-$C_4$ intervenant dans la définition de Z, on peut mentionner les restes hydrocarbonés ayant au plus quatre atomes de carbone à chaîne linéraire ou ramifiée tels que $CH_3$, $CH_2CH_3$, $CH_2CH_2CH_3$, $CH_2CH_2CH_2CH_3$, $CH(CH_3)_2$ et $C(CH_3)_3$.

Par sels d'addition, on entend ici les sels d'addition d'acide obtenus par réaction d'une base libre de formule I avec un acide minéral ou organique, d'une part, et les sels d'ammonium, d'autre part. Parmi les acides utilisables pour salifier la base libre de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide, tels que notamment les chlorhydrates, sont préférés aux sels d'ammonium.

L'invention concerne en particulier la 1-(4-aminophényl)-2-(3-méthylpipéridino)propanone, la 1-(4-acétylaminophényl)-2-(3-méthylpipéridino)propanone, la 1-(4-amino-3,5-dichlorophényl)-2-(3-méthylpipéridino)propanone, la 1-(4-acétylamino-3,5-dichlorophényl)-2-(3-méthylpipéridino)propanone et leurs sels d'addition.

Les composés où A=B=Cl sont préférés.

Un certain nombre de composés selon l'invention a été consigné de façon nullement limitative dans le tableau I ci-après, avec, pour comparaison, les deux homologues CP-1 et CP-2 décrits dans les documents précités, qui sont des agents antidépresseurs et stimulants du SNC mais qui ne présentent pas d'effets cardiovasculaires.

**TABLEAU I**

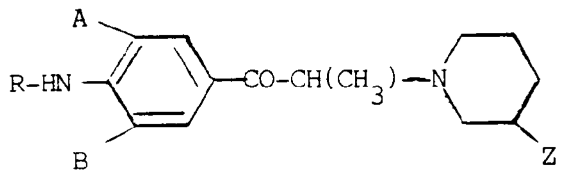

(I)

| Exemple | No de Code | R | A | B | Z |
|---|---|---|---|---|---|
| Ex 1 (a) | CRL 41 419 | $CH_3CO$ | 3-Cl | 5-Cl | $CH_3$ |
| Ex 2 (a) | CRL 41 418 | H | 3-Cl | 5-Cl | $CH_3$ |
| Ex 3 (b) | CRL 41 419A | $CH_3CO$ | 3-Cl | 5-Cl | $CH_3$ |
| Ex 4 (a) | CRL 41 416 | $CH_3CO$ | H | H | $CH_3$ |
| Ex 5 (c) | - | H | H | H | $CH_3$ |
| CP-1(c,d) | CRL 41 241 | H | H | H | H |
| CP-2(a,e) | CRL 41 240 | $CH_3CO$ | H | H | H |

**Notes**

(a) : monochlorhydrate ;
(b) : méthanesulfonate ;
(c) : dichlorhydrate ;
(d) : décrit à l'exemple 11 de EP-A-0 174 242 ;
(e) : décrit à l'exemple 11 de EP-A-0 138 714.

Les composés de formule I peuvent être préparés selon une méthode connue en soi par application de mécanismes réactionnels classiques.

En particulier ils peuvent être synthétisés suivant les méthodes opératoires décrites dans les documents de brevet précités EP-A-0 174 242 et EP-A-0 138 714.

Le procédé que l'on préconise ici consiste à faire réagir une 3-alkylpipéridine de formule

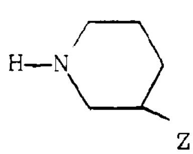

(II)

dans laquelle Z est un groupe alkyle en $C_1$-$C_4$ comme défini ci-dessus, avec une 1-(4-aminophényl)-2-halo-

génopropanone de formule

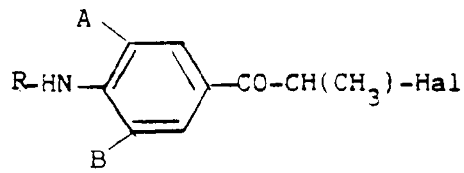

(III)

dans laquelle R, A et B sont définis comme indiqué ci-dessus et Hal représente un atome d'halogène (notamment F, Cl et Br, l'atome d'halogène préféré étant ici le chlore).

La réaction II + III s'applique à la synthèse de tous les composés de formule I ci-dessus. De façon avantageuse, on utilisera 0,2 à 0,3 mole du composé III pour 1 mole du composé de formule II, pendant au moins 0,5 h, à une température comprise entre la température ambiante (15-25°C) et la température de reflux du milieu réactionnel. Dans cette réaction, le composé II intervient soit comme solvant soit encore mieux comme co-solvant.

En variante, chaque composé de formule I, où R = $CH_3CO$, peut être préparé par acétylation du composé correspondant de formule I, où R = H, selon une méthode d'acétylation connue en soi. Dans cette optique, on fera réagir 0,2 à 0,4 mole de composé de formule I où R = H, avec 1 mole d'halogénure d'acétyle, de préférence le chlorure d'acétyle (l'atome de chlore étant l'atome d'halogène préféré comme indiqué ci-dessus). Dans cette variante, l'acide acétique intervient en général comme solvant.

Les composés selon l'invention présentent des propriétés thérapeutiques bénéfiques. Ils agissent en particulier au niveau cardiovasculaire et présentent des effets vasodilatateurs inattendus eu égard à l'enseignement des brevets européens précités EP-A-0 138 714 et EP-A-0 174 242, dès lors que les produits de comparaison CP-1 et CP-2 sont dépourvus d'effets cardiovasculaires et agissent essentiellement en tant que antidépresseurs du SNC.

Les composés les plus intéressants selon l'invention en tant qu'agents vasodilatateurs sont la 1-(4-amino-3,5-dichlorophényl)-2-(3-méthylpipéridino)propanone, la 1-(4-acétylamino-3,5-dichlorophényl)-2-(3-méthylpipéridino)propanone et leurs sels d'addition non-toxiques.

Selon l'invention on préconise une composition thérapeutique qui est caractérisée en ce qu'elle renferme, en association avec un excipient physiologiquement acceptable, au moins un dérivé choisi parmi les composés de formule I et leurs sels d'addition non-toxiques.

Bien entendu, dans une telle composition le principe actif, à savoir le composé de formule I ou l'un de ses sels non-toxiques, intervient en quantité pharmaceutiquement efficace.

Selon l'invention, on préconise l'utilisation d'une substance choisie parmi l'ensemble comprenant (i) la 1-(4-amino-3,5-dichlorophényl)-2-(3-méthylpipéridino)propanone, (ii) la 1-(4-acétylamino-3,5-dichlorophényl)-2-(3-méthylpipéridino)propanone, et, (iii) leurs sels d'addition non-toxiques, pour l'obtention d'un médicament vasodilatateur destiné à une utilisation en thérapeutique en thérapeutique humaine vis-à-vis des maladies du système cardiovasculaire, notamment les maladies telles que l'infarctus qui impliquent l'administration d'un vasodilatateur coronarien, d'une part, et les troubles circulatoires du cerveau et des extrémités qui impliquent l'administration d'un vasodilatateur périphérique, d'autre part.

D'autres avantages et caractéristiques de l'invention seront mieux compris à la lecture qui va suivre d'exemples de préparation et de résultats d'essais pharmacologiques.

## PREPARATION I

Obtention du monochlorhydrate de 1-(4-amino-3,5-dichlorophényl)-2-(3-méthylpipéridino)propanone

(Exemple 2 ; No de Code : CRL 41 418)
autre nomenclature : monochlorhydrate d'α-(3-méthylpipéridino)-4-amino-3,5-dichloropropiophénone

a) 1-(4-acétylaminophényl)-2-chloropropanone

Au sein d'un mélange de 69,2 g (0,50 mole) d'acétanilide et de 205 g (1,50 moles) de chlorure d'aluminium dans 525 ml de sulfure de carbone, on coule en 1,5 h une quantité de 118 g (0,93 mole) de chlorure de 2-chloropropionyle et on chauffe au reflux pendant 1 h. On décante le surnageant, et on hydrolyse le résidu par 1900 ml d'eau glacée et 385 ml d'acide chlorhydrique 4N. Le précipité formé est isolé par filtration, repris par du benzène que l'on distille azéotropiquement au moyen d'un dispositif Dean-Stark. Après traitement de la solution chaude au noir de carbone (CXA) et refroidissement, on isole 102,4 g (rendement : 90,82 %) du produit attendu sous la forme d'une poudre légèrement beige.

$F_{inst.}$(Kofler) = 120°C.

b) 1-(4-aminophényl)-2-chloropropanone

On porte au reflux pendant 1,5 h un mélange de 102 g (0,45 mole) de 1-(4-acétylaminophényl)-2-chloropropanone dans 400 ml d'acide HCl 6N. On traite la solution résultante encore chaude avec du noir de carbone et neutralise avec de l'ammoniaque. Le précipité formé est isolé par filtration et repris par du benzène que l'on distille azéotropiquement. Après traitement de la solution restante encore chaude au moyen de noir de carbone, on laisse cristalliser et on recueille par filtration 65 g (rendement : 78,7 %) du produit attendu qui se présente sous la forme d'une poudre de couleur beige.

$F_{inst.}$(Kofler) = 100 - 102°C.

c) 1-(4-amino-3,5-dichlorophényl)-2-chloropropanone

Au sein d'une solution dans 180 ml de tétrachlorure de carbone, maintenue à 70°C, de 33 g (0,18 mole) de 1-(4-aminophényl)-2-chloropropanone, on introduit par fractions pendant 1 h une quantité de 72 g (0,54 mole) de N-chlorsuccinimide. On dilue le mélange réactionnel au moyen de 200 ml de chloroforme, élimine l'insoluble formé par filtration, puis amène le filtrat à siccité sous pression réduite. Le résidu d'évaporation ainsi obtenu est repris avec du diéthyléther et on écarte l'insoluble, qui en résulte, par filtration ainsi que le solvant par distillation. La masse brune restante est purifiée par recristallisation de l'isopropanol. On obtient 30,8 g (rendement : 67,8 %) du produit attendu qui se présente sous la forme d'une poudre blanche.

$F_{inst.}$(Kofler) = 110 - 112°C.

d) CRL 41 418

On agite 0,5 h à la température ambiante (15-20°C) puis 0,5 h à 70°C, un mélange de 25,25 g (0,10 mole) de 1-(4-amino-3,5-dichlorophényl)-2-chloropropanone et de 50 g (0,50 mole) de 3-méthylpipéridine dans 60 ml d'eau. On dilue le mélange réactionnel au moyen de 100 ml d'eau, extrait le milieu résultant avec de l'acétate d'éthyle puis extrait la phase acétate d'éthyle au moyen d'une solution aqueuse d'HCl diluée. Après neutralisation de la phase aqueuse ainsi obtenue, par l'ammoniaque, on extrait avec du diéthyléther puis traite la solution éthérée avec de l'éthanol contenant de l'acide HCl. Il se forme un insoluble huileux que l'on soumet à une opération de lavage au moyen d'acétone puis à une purification par recristallisation du mélange acétonitrile/isopropanol (4/1) v/v. On obtient ainsi 19,6 g (rendement : 55,8 %; rendement global des étapes a-d : 27 %) du produit attendu qui se présente sous la forme d'une poudre beige qui est soluble dans l'eau à la concentration de 50 g/l.

F = 200 - 220°C (avec décomposition).

PREPARATION II

Obtention du monochlorhydrate de 1-(4-acétylamino-3,5-dichlorophényl)-2-(3-méthylpipéridino)propanone

(Exemple 1; No. de Code : CRL 41 419)

autre nomenclature : monochlorhydrate d'α-(3-méthylpipéridino)-4-acétamido-3,5-dichloropropiophénone.

On agite pendant une nuit, à la température ambiante une solution comprenant 13,5 g (0,035 mole) de monochlorhydrate de 1-(4-amino-3,5-dichlorophényl)-2-(-méthylpipéridino)propanone et 8,2 ml (0,114 mole) de chlorure d'acétyle dans 50 ml d'acide acétique. On évapore à sec le milieu réactionnel sous pression réduite et purifie le résidu d'évaporation ainsi obtenu par lavage dans l'acétone. On obtient 15 g (rendement : 100 % environ) du produit attendu qui se présente sous la forme d'une poudre de couleur légèrement beige.

F = 220°C (avec décomposition).

PREPARATION III

Autre mode d'obtention du monochlorhydrate de 1-(4-acétylamino-3,5-dichlorophényl)-2-(3-méthylpipéridino)propanone

(Exemple 1; No. de Code : CRL 41 419)

En procédant comme indiqué à la Préparation Id, mais en remplaçant la 1-(4-amino-3,5-dichlorophényl)-2-chloropropanone par la 1-(4-acétylamino-3,5-dichlorophényl)-2-chloropropanone, on obtient le CRL 41 419.

F = 220°C (avec décomposition).

PREPARATION IV

Obtention du monochlorhydrate de 1-(4-acétylaminophényl)-2-(3-méthylpipéridino)propanone

Exemple 4; No. de Code : CRL 41 416)

On agite pendant 24 h, à la température ambiante un mélange comprenant 60 g (0,266 mole) de 1-(4-acétylaminophényl)-2-chloropropanone [produit obtenu selon le procédé de la Préparation Ia], 100 g (1,0 mole) de 3-méthylpipéridine et 100 ml d'eau. On dilue le milieu réactionnel avec 250 ml d'eau et extrait l'insoluble au moyen d'acétate d'éthyle. On lave la phase acétate d'éthyle avec 3 x 500 ml d'eau. On sèche la phase acétate d'éthyle sur sulfate de sodium anhydre puis ajoute sous agitation de l'éthanol contenant de l'acide chlorhydrique. Le précipité formé est recueilli par filtration et purifié par recristallisation du mélange isopropanol/éthanol (5/4) v/v pour donner 66,3 g (rendement : 76,8 %) du produit attendu qui se présente sous la forme d'une poudre blanche soluble dans l'eau.

F = 230°C (avec décomposition).

On a résumé ci-après les résultats des essais toxicologiques, neuropsychopharmacologiques et cardiovasculaires qui ont été entrepris avec les composés selon l'invention.

A. ESSAIS RELATIFS AU CRL 41 419 (PRODUIT DE L'EXEMPLE 1)

- ETUDE TOXICOLOGIQUE

Dans les études toxicologique et neuropyschopharmacologique qui suivent, le CRL 41 419 en solution dans de l'eau distillée a été administré par voie intrapéritonéale sous un volume de 20 ml/kg chez la souris mâle et de 5 ml/kg chez le rat mâle. Le pH de la solution varie en fonction de la concentration en CRL 41 419; il passe de 4,5 pour une concentration de 50 g/l, à 5 pour une concentration de 12,5 g/l et à 5,5 pour des concentrations inférieures ou égales à 6,4 g/l.

**TOXICITE**

Chez la souris mâle la DL-0 (dose maximale non mortelle) par voie intrapéritonéale est supérieure à 256 mg/kg et la DL-100 (dose minimale mortelle pour tous les animaux traités) est de l'ordre de 512 mg/kg (à cette dose la mort des souris intervient dans les 0,5-4 h qui suivent l'administration du CRL 41 419).

- ETUDE NEUROPSYCHOPHARMACOLOGIQUE

**COMPORTEMENT GLOBAL ET REACTIVITES**

Des lots de trois animaux sont observés avant, puis 0,25 h, 0,50 h, 1 h, 2 h, 3 h et 24 h après administration du CRL 41 419. On constate :

1°) chez la souris

aux doses de 2 mg/kg, 4 mg/kg et 8 mg/kg :

- un comportement et des réactivités sensiblement comparables à ceux du lot témoin;

à la dose de 32 mg/kg :

- une hypothermie (-1°C) pendant 2 heures; à la dose de 128 mg/kg :
  - une sédation pendant 0,5 h;
  - une hypothermie pendant 3 h (valeur maximale : -3,7°C, 30 minutes après administration);
  - une diminution de la réactivité au toucher, du tonus musculaire et de la réaction d'agressivité; et,

2°) chez le rat

aux doses de 1 mg/kg, 2 mg/kg, 4 mg/kg et 16 mg/kg :

- un comportement, des réactivités, une variation de la température rectale et du diamètre pupillaire sensiblement comparables à ceux du lot témoin ;

à la dose de 64 mg/kg :

- une sédation pendant 0,25 h accompagnée d'une diminution de la fréquence cardiaque.

- ETUDE CARDIOVASCULAIRE

Dans l'étude cardiovasculaire, le CRL 41 419 a été administré en solution dans du soluté isotonique de chlorure de sodium (concentration en NaCl : 9 g/l dans de l'eau), à pH 3,3, la concentration maximale dudit

CRL 41 419 ayant été utilisée étant de 66 g/l.

I. **ADMINISTRATION PAR VOIE INTRADUODENALE**

Trois chiens mâles ayant un poids moyen de 13,6 kg anesthésiés au nembutal reçoivent le CRL 41 419 par voie intraduodénale aux doses successives de 0 mg/kg (chaque animal servant de témoin par rapport à lui-même); 0,55 mg/kg; 1,1 mg/kg; 2,75 mg/kg; 5,5 mg/kg; 11 mg/kg; et respectivement 22 mg/kg [doses correspondant à 0; 0,5 mg/kg; 1 mg/kg; 2,5 mg/kg; 5 mg/kg; 10 mg/kg; et respectivement 20 mg/kg en 1-(4-acétylamino-3,5-dichlorophényl)-2-(3-méthylpipéridino)propanone, base libre du CRL 41 419].

On mesure la pression artérielle, les fréquences cardiaque et respiratoire, le débit artériel fémoral, le débit artériel vertébral et les températures rectale et cutanée. On observe la coloration de la peau. On dose également les gaz sanguins chez un des trois chiens.

On constate que le CRL 41 419 administré aux doses successives de 0,55 à 22 mg/kg par voie I.D.

- augmente :
  - les débits fémoraux chez 2 chiens sur 3 à partir de la dose de 5,5 mg/kg, et chez chiens sur 3 à la dose de 11 mg/kg,
  - le débit vertébral chez 2 chiens sur 3 à partir de la dose de 11 mg/kg,
  - la fréquence cardiaque à la dose de 22 mg/kg,
  - les températures rectale (+ 1,2°C) et cutanée (+ 1,5°C),
  - l'offre en $O_2$ dissous à la dose de de 22 mg/kg, et
  - la production de $CO_2$ à la dose de 22 mg/kg;
- diminue :
  - les résistances vasculaires vertébrales et fémorales,
  - la fréquence respiratoire, et
  - les pH artériel et veineux surtout à la dose de 22 mg/kg;
- ne modifie pas :
  - la pression artérielle; et,
- provoque :
  - un rosissement de la peau à partir de la dose de 22 mg/kg.

II. **ADMINISTRATION PAR VOIE INTRAVEINEUSE**

Les trois chiens utilisés ci-dessus reçoivent à la fin des essais du point I ci-dessus entrepris par administration I.D., une dose unique de 4,4 mg/kg de CRL 41 419 par voie intraveineuse. On constate que par administration I.V. le CRL 41 419

- augmente :
  - les débits fémoraux (variation moyenne de + 189 %) chez les 3 chiens,
  - la fréquence cardiaque,
  - le débit vertébral (variation moyenne de + 94 % chez 2 chiens sur 3), et
  - les températures rectale et cutanée;
- diminue :
  - les résistances fémorale et vertébrale, et
  - la fréquence respiratoire; et
- ne modifie pas :
  - la pression artérielle, et
  - la coloration de la peau.

III. **ACTION SUR LA PRESSION DE PERFUSION DU TRAIN POSTERIEUR DE RAT**

On a évalué l'action du CRL 41 419 sur la pression de perfusion du train postérieur de rat.

Des lots de 10 rats mâles adultes chacun (un lot témoin et un lot par dose de produit à tester) sont soumis au protocole opératoire suivant. On maintient la température rectale au moyen d'une lampe entre 36,5°C et 37,5°C de rats anesthésiés au nembutal, et on mesure la pression de perfusion du train postérieur, perfusé à travers l'aorte abdominale au dessous des reins, à débit constant (5 ml/min) avec un liquide nutritif aéré et réchauffé. Chaque animal est ensuite sacrifié par injection intraveineuse de KCl. On évalue l'action d'une gamme de 4 injections intraveineuses de 0,1 ml de soluté isotonique de NaCl à 9 g/l ou de 4 doses croissantes de CRL 41 419 administré par voie I.A. (non cumulées) en solution dans de l'eau distillée, sur la pression de perfusion augmentée par perfusion continue dans le liquide, de chlorhydrate de noradrénaline (9 microgrammes/minute).

Les traitements (NaCl à 9 g/l ou CRL 41 419) sont effectués au hasard sur les deux lots d'animaux traités.

On constate que, à toutes les doses qui ont été utilisées (0,01 à 10 mg), le CRL 41 419 diminue la pression de perfusion du train postérieur de rat alors que ladite pression avait été augmentée par le chlorhydrate de noradrénaline. La diminution de la pression de perfusion est statistiquement significative par rapport (i) aux effets du NaCl à 9 g/l, et (ii) par rapport au lot témoin.

On constate par ailleurs que l'intensité et la durée d'action du CRL 41 419 augmentent proportionnellement aux doses utilisées. Après l'administration I.A. de 10 mg de CRL 41 419, le temps dit de demi-retour est de 435 secondes.

L'ensemble de ces résultats met en évidence les propriétés vasodilatatrices du CRL 41 419 qui sont objectivées notamment par l'augmentation de l'offre en $O_2$, d'une part, et la non-modification des pressions artérielles dans les essais I et II ci-dessus, d'autre part.

B.ESSAIS RELATIFS AU CRL 41 418 (PRODUIT DE L'EXEMPLE 2)

Les études toxicologique et neuropsychopharmacologique du CRL 41 418 ont été entreprises selon les modalités opératoires décrites ci-dessus pour le CRL 41 419, le CRL 41 418 à étudier étant administré en solution dans de l'eau distillée, à pH 5,5, par voie intrapéritonéale, sous un volume de 20 ml/kg chez la souris mâle et de 5 ml/kg chez le rat mâle.

- ETUDE TOXICOLOGIQUE

**TOXICITE**

La DL-O du CRL 41 418 est supérieure à 128 mg/kg et la DL-100 dudit CRL 41 418 est de l'ordre de 220 mg/kg par voie intrapéritonéale.

- ETUDE NEUROPSYCHOPHARMACOLOGIQUES

**COMPORTEMENT GLOBAL ET REACTIVITES**

On observe

1°) chez la souris

aux doses de 1 mg/kg, 4 mg/kg et 16 mg/kg:

- un comportement et des réactivités sensiblement comparables à ceux du lot témoin;

à la dose de 64 mg/kg :

- une sédation;
- une diminution de la fréquence respiratoire pendant 0,5 h,
- une hypothermie pendant 3 h (variation maximale de - 2,8°C, 30 minutes après administration du CRL 41 418); et
- une diminution de la fréquence respiratoire; et,

2°) chez le rat

aux doses de 0,5 mg/kg, 2 mg/kg et 8 mg/kg :

- un comportement, des réactivités, une variation de la température rectale et du diamètre pupillaire sensiblement comparables à ceux du lot témoin ; et,

à la dose de 32 mg/kg :

- une mydriase modérée pendant 1 h,
- une hypothermie pendant 1 h (variation maximale : - 1,2°C, 30 minutes après administration du CRL 41 418); et,
- une diminution de la réactivité au toucher et du tonus musculaire pendant 0,5 h.

- ETUDE CARDIOVASCULAIRE

Dans l'étude cardiovasculaire le CRL 41 418 a été administré par voie I.D. en solution dans du soluté de NaCl à 9 g/l (concentration maximale en CRL 41 418 utilisée : 62 g/l, à pH 3).

Trois chiens mâles (poids moyen : 12,1 kg chacun) anesthésiés au nembutal reçoivent le CRL 41 418 par voie intraduodénale, aux doses successives de 0 (chaque animal servant de contrôle vis-à-vis de lui-même); 0,5 mg/kg; 1 mg/kg; 2,5 mg/kg; 10 mg/kg et 20 mg/kg. Le protocole opératoire est celui indiqué ci-dessus pour le CRL 41 419.

On constate que, par voie I.D., le CRL 41 418 administré aux doses de 0,5 à 20 mg/kg

- augmente :
  - la fréquence cardiaque à partir de la dose de 2,5 mg/kg,
  - le débit vertébral chez les 3 chiens à partir de de la dose de 2,5 mg/kg,
  - la température cutanée fortement, et, la température rectale très modérément,
  - la pression différentielle,
  - la pression systolique,
  - la fréquence respiratoire,
  - l'offre en $O_2$ au niveau vertébral, et, beaucoup moins la consommation en $O_2$ (chez 2 chiens sur 3) estimée au niveau général;
- ne modifie pas :
  - les pressions artérielles diastolique et moyenne.

Vis-à-vis de l'isoprénaline (administrée par voie intraveineuse à la dose de 1 μg/kg), le CRL 41 418, administré par voie I.D. à la dose cumulée de 39 mg/kg I.D.

- diminue : (i.e. s'oppose partiellement à) :
  - l'hypotension diastolique induite par l'isoprénaline [variation de + 40 mmHg (i.e. environ 4,33 x $10^3$Pa) à + 76 mmHg (i.e. environ 9,33 x $10^4$Pa) de la pression diastolique], et
- ne diminue pas :
  - globalement la tachycardie induite par l'isoprénaline [on n'observe seulement qu'une diminution de la variation de l'ordre de - 23 battements/minute à - 68 battements/minute].

Vis-à-vis de la noradrénaline (administrée à la dose de 2 μg/kg par voie intraveineuse), on constate que le CRL 41 418, à la dose cumulée de 39 mg/kg I.D.

- réduit :
  - l'hypertension systolique induite par la noradrénaline.

Par ailleurs, le propranolol (1 mg/kg I.V.) perfusé en 6 minutes en fin d'expérience, diminue la pression artérielle et la fréquence cardiaque de 2 chiens sur 2 et ne diminue que le débit vertébral d'un chien sur deux.

Les résultats de ces essais mettent en évidence que le CRL 41 418 agit en tant que vasodilatateur. La tachycardie observée est due à une stimulation des récepteurs béta-adrénergiques car elle est supprimée par le propranolol.

C. ESSAIS COMPARATIFS AVEC CRL 41 240 et CRL 41 241

Des essais chez le chien anesthésié analogues à ceux décrits pour le CRL 41419 ont été réalisés avec le CRL 41 240 et le CRL 41 241.

Administrés par voie I.D. à des doses de 0,1 à 20 mg/kg, les CRL 41 240 et 41 241 se sont avérés dépourvus d'effet sur les débits vertébraux et fémoraux.

En clinique on a obtenu de bons résultats en administrant chez l'homme adulte le CRL 41 419 en tant qu'agent vasodilatateur coronarien, d'une part, et en tant qu'agent vasodilatateur périphérique, d'autre part. En particulier, le CRL 41 419 s'est avéré être un excellent vasodilatateur à la dose quotidienne de 200 à 300 mg (répartie en deux à trois prises de chacune 100 mg, sous forme de comprimés ou gélules), notamment chez les patients souffrant de troubles circulatoires au niveau du cerveau et au niveau des extrémités.

## Revendications

### Revendications pour les Etats contractants suivants : AT, BE, CH, DE, DK, FR, GB, GR, IT, LI, LU, NL, SE

**1.** Composé choise parmi :

(a) les composés répondant à la formule générale

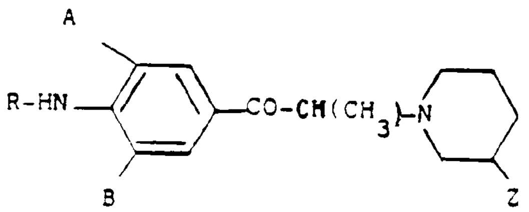

(I)

où R est H ou $CH_3CO$, A est H ou Cl, B indépendamment de A est H ou Cl, et Z est un groupe alkyle en $C_1$-$C_4$; et,
(b) leurs sels d'addition.

2. Composé selon la revendication 1, caractérisé en ce que A = B = Cl.

3. Composé selon la revendication 1, caractérisé en ce que R = H ou $CH_3CO$, A = B = Cl, et Z = $CH_3$.

4. 1-(4-Acétylamino-3,5-dichlorophényl)-2-(3-méthylpipéridino)propanone et ses sels d'addition, selon la revendication 1.

5. 1-(4-Amino-3,5-dichlorophényl)-2-(3-méthylpipéridino) propanone et ses sels d'addition, selon la revendication 1.

6. Composition thérapeutique caractérisée en ce qu'elle renferme, en association avec un excipient physiologiquement acceptable, au moins un composé choisi parmi les composés de formule I suivant la revendication 1 et leurs sels d'addition non-toxiques.

7. Utilisation d'une substance choisie parmi :
(i) la 1-(4-acétylamino-3,5-dichlorophényl)-2-(3-méthylpipéridino) propanone, (ii) la 1-(4-amino-3,5-dichlorophényl)-2-(3-méthylpipéridino)propanone et (iii) leurs sels d'addition non-toxiques, pour l'obtention d'un médicament vasodilatateur destiné à une utilisation en thérapeutique humaine vis-à-vis des troubles de la circulation.

8. Utilisation selon la revendication 7 , caractérisée en ce que l'on utilise une substance choisie parmi l'ensemble comprenant la 1-(4-acétylamino-3,5-dichlorophényl)-2-(3-méthylpipéridino)propanone et ses sels d'addition non-toxiques, pour l'obtention d'un médicament vasodilatateur périphérique destiné à une utilisation en thérapeutique humaine vis-à-vis des troubles circulatoires du cerveau et des extrémités.

9. Procédé de préparation d'un composé de formule I selon la revendication 1 et de ses sels d'addition, ledit procédé étant caractérisé en ce qu'il consiste à faire réagir une 3-alkylpipéridine de formule

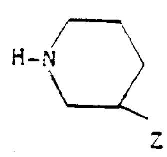

(II)

dans laquelle Z est un groupe alkyle en $C_1$-$C_4$ comme dans la revendication 1, avec un composé 1-(4-aminophényl)-2-halogénopropanone de formule

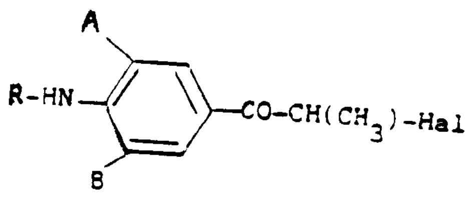

(III)

dans laquelle R, A et B sont définis comme dans la revendication 1 et Hal représente un atome d'halogène.

**Revendications pour l'Etat contractant suivant : ES**

1. Procédé de préparation d'un composé choisi parmi :
   (a) les composés répondant à la formule générale

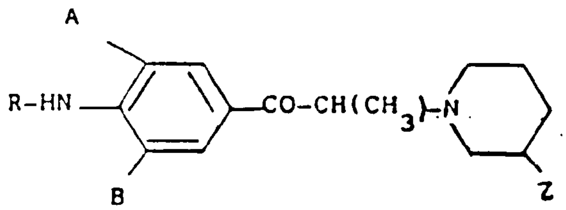

(I)

où R est H ou $CH_3CO$, a est H ou Cl, B indépendamment de A est H ou Cl, et Z est un groupe alkyle en $C_1$-$C_4$; et
(b) leurs sels d'addition,
ledit procédé étant caractérisé en ce qu'il consiste à faire réagir une 3-alkylpipéridine de formule

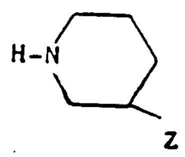

(II)

dans laquelle Z est un groupe alkyle en $C_1$-$C_4$ comme défini ci-dessus, avec un composé 1-(4-aminophényl)-2-halogénopropanone de formule

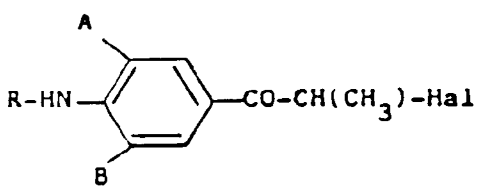

(III)

dans laquelle R, A et B sont définis comme indiqué ci-dessus et Hal représente un atome d'halogène.

2. Procédé selon la revendication 1, caractérisé en ce que A = B = Cl.

3. Procédé selon la revendication 1, caractérisé en ce que R = H ou $CH_3CO$, A = B = Cl, et Z = $CH_3$.

**Patentansprüche**

**Patentansprüche für folgende Vertragsstaaten : AT, BE, CH, DE, DK, FR, GB, GR, IT, LI, LU, NL, SE**

1. Verbindung, ausgewählt aus:
   (a) den Verbindungen der allgemeinen Formel

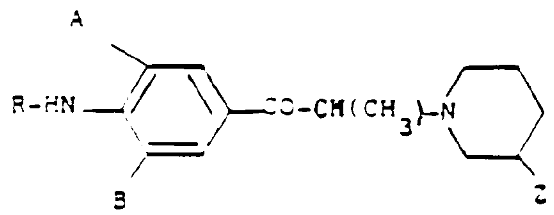

(I)

in der R gleich H oder $CH_3CO$, A gleich H oder Cl, B unabhängig von A gleich H oder Cl und Z eine $C_1$ -$C_4$-Alkylgruppe ist, und
(b) ihren Additionssalzen.

2. Verbindung nach Anspruch 1, dadurch gekennzeichnet, daß A = B = Cl.

3. Verbindung nach Anspruch 1, dadurch gekennzeichnet, daß R = H oder $CH_3CO$, A = B = Cl und Z = $CH_3$ ist.

4. 1-(4-Acetylamino-3,5-dichlorphenyl)-2-(3-methylpiperidino) propanon und seine Additionssalze gemäß Anspruch 1.

5. 1-(4-Amino-3,5-dichlorphenyl)-2-(3-methylpiperidino)propanon und seine Additionssalze gemäß Anspruch 1.

6. Therapeutische Zusammensetzung, dadurch gekennzeichnet, daß sie in Verbindung mit einem physiologisch akzeptablen Bindemittel wenigstens eine Verbindung enthält, die unter den Verbindungen der Formel I nach Anspruch 1 und ihren nichttoxischen Additionssalzen ausgewählt ist.

7. Verbindung einer Substanz, ausgewählt aus:
(i) 1-(4-Acetylamino-3,5-dichlorphenyl)-2-(3-methylpiperidino) propanon, (ii) 1-(4-Amino-3,5-dichlorphenyl)-2-(3-methylpiperidino) propanon und (iii) ihren nichttoxischen Additionssalzen zur Bildung eines gefäßerweiternden Arzneimittels, das in der Humantherapie zur Verwendung bei Kreislaufstörungen bestimmt ist.

8. Verwendung nach Anspruch 7, dadurch gekennzeichnet, daß eine Substanz, die aus der Gruppe ausgewählt ist, die 1-(4-Acetylamino-3,5-dichlorphenyl)-2-(3-methylpiperidino)propanon und seine nichttoxischen Additionssalze umfaßt, zur Bildung eines peripheren gefäßerweiternden Arzneimittels verwendet wird, das in der Humantherapie zur Verwendung bei Kreislaufstörungen des Gehirns und der Extremitäten bestimmt ist.

9. Verfahren zur Herstellung einer Verbindung der Formel I nach Anspruch 1 und ihrer Additionssalze, dadurch gekennzeichnet, daß man ein 3-Alkylpiperidin der Formel II

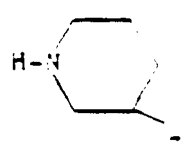

(II)

in der Z eine $C_1$-$C_4$-Alkylgruppe wie in Anspruch 1 ist, mit einer 1-(4-Aminophenyl)-2-halogenpropanon-Verbindung der Formel III

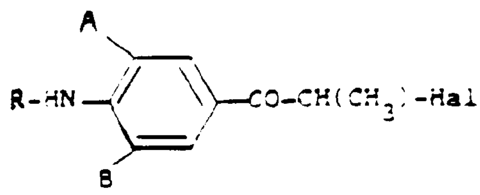

(III)

reagieren läßt, in der R, A und B die in Anspruch 1 angegebenen Bedeutungen haben und Hal ein Halogenatom darstellt.

**Patentansprüche für folgenden Vertragsstaat : ES**

1. Verfahren zur Herstellung einer Verbindung, die ausgewählt ist aus:
   (a) den Verbindungen der allgemeinen Formel

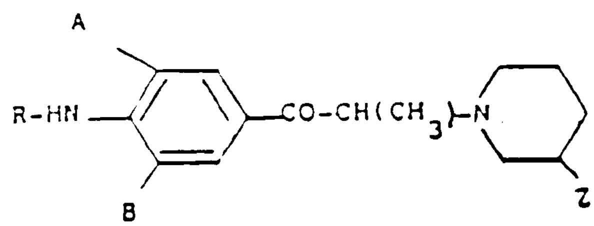

(I)

in der R gleich H oder $CH_3CO$, A gleich H oder Cl, B unabhängig von A gleich H oder Cl und Z eine $C_1$-$C_4$-Alkylgruppe ist, und
(b) ihren Additionssalzen,
dadurch gekennzeichnet, daß man ein 3-Alkylpiperidin der Formel

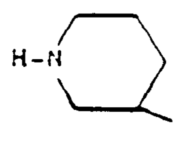

(II)

in der Z eine $C_1$-$C_4$-Alkylgruppe gemäß der vorstehenden Definition ist, mit einer 1-(4-Aminophenyl)-2-halogenpropanon-Verbindung der Formel

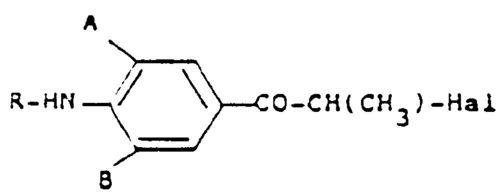

(III)

reagieren läßt, in der R, A und B die oben angegebenen Bedeutungen haben und Hal ein Halogenatom darstellt.

2. Verfahren nach Anspruch 1, dadurch gekennzeichnet, daß A = B = Cl.

3. Verfahren nach Anspruch 1, dadurch gekennzeichnet, daß R = H oder $CH_3CO$, A = B = Cl und Z = $CH_3$ ist.

## Claims

**Claims for the following Contracting States : AT, BE, CH, DE, GB, GR, IT, LI, LU, NL, SE**

1. Compound selected from:
(a) the compounds corresponding to the general formula

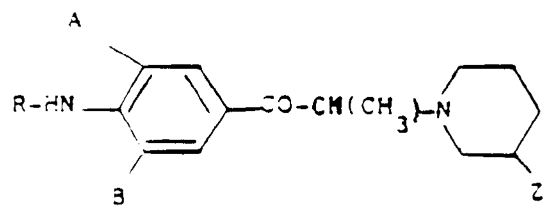

(I)

where R is H or $CH_3CO$, A is H or Cl, B, independently of A, is H or Cl and Z is a $C_1$-$C_4$-alkyl group; and (b) their addition salts.

2. Compound according to Claim 1, characterized in that A = B = Cl.

3. Compound according to Claim 1, characterized in that R = H or $CH_3CO$, A = B = Cl and Z = $CH_3$.

4. 1-(4-acetylamino-3,5-dichlorophenyl)-2-(3-methylpiperidino)propanone and its addition salts, according to Claim 1.

5. 1-(4-amino-3,5-dichlorophenyl)-2-(3-methylpiperidino)propanone and its addition salts, according to Claim 1.

6. Therapeutic composition, characterized in that it includes, in association with a pharmaceutically acceptable excipient, at least one compound selected from the compounds of formula I, according to Claim 1, and their non-toxic addition salts.

7. Use of a substance selected from:
(i) 1-(4-acetylamino-3,5-dichlorophenyl)-2-(3-methylpiperidino)propanone, (ii) 1-(4-amino-3,5-dichlorophenyl)-2-(3-methyl-piperidino)propanone and (iii) their non-toxic addition salts, for obtaining a vasodilatory medicament intended for use in human therapy for circulation disorders.

8. Use according to Claim 7, characterized in that a substance selected from the group comprising 1-(4-acetylamino-3,5-dichlorophenyl)-2-(3-methyl-piperidino)propanone and its non-toxic addition salts is used for obtaining a peripheral vasodilatory medicament intended for use in human therapy for circulation disorders of the brain and the extremities.

9. Process for the preparation of a compound of formula I according to Claim 1 and of its addition salts, the said process being characterized in that it consists in reacting a 3-alkylpiperidine of formula

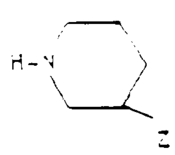

(II)

in which Z is a $C_1$-$C_4$-alkyl group as in Claim 1, with a 1-(4-aminophenyl)-2-halogenopropanone compound of formula

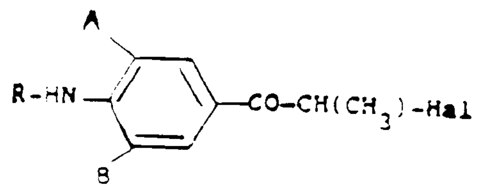

(III)

in which R, A and B are defined as in Claim 1 and Hal represents a halogen atom.

**Claims for the following Contracting State : ES**

1. Process for the preparation of a compound selected from:
(a) the compounds corresponding to the general formula

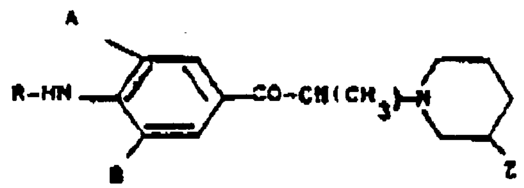

(I)

where R is H or $CH_3CO$, A is H or Cl, B, independently of A, is H or Cl and z is a $C_1$-$C_4$-alkyl group; and
(b) their addition salts,
the said process being characterized in that it consists in reacting a 3-alkylpiperidine of formula

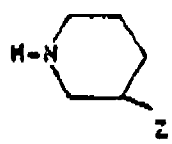

(II)

in which z is a $C_1$-$C_4$-alkyl group as defined above, with a 1-(4-aminophenyl)-2-halogenopropanone compound of formula

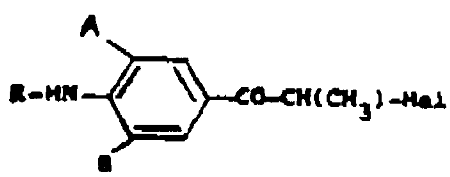

(III)

in which R, A and B are defined as indicated above and Hal represents a halogen atom.

2. Process according to Claim 1, characterized in that A = B = Cl.

3. Process according to Claim 1, characterized in that R = H or $CH_3CO$, A = B = Cl and z = $CH_3$.